Europäisches Patentamt
European Patent Office
Office européen des brevets

(19)

(11) Publication number: **0 446 722 A1**

(12) **EUROPEAN PATENT APPLICATION**

(21) Application number: **91103085.6**

(22) Date of filing: **01.03.91**

(51) Int. Cl.5: **C07C 43/295**, C07C 49/84, C07C 317/22, C07C 323/20

(30) Priority: **08.03.90 IL 93687**
**20.02.91 IL 97303**

(43) Date of publication of application:
**18.09.91 Bulletin 91/38**

(84) Designated Contracting States:
**BE CH DE ES FR GB IT LI NL**

(71) Applicant: **Bromine Compounds Ltd.**
**Makleff House P.O.B. 180**
**Beer-Sheva 84101(IL)**

(72) Inventor: **Oren, Jacob**
**8/9, Reuven Str.**
**Qiryat Bialik(IL)**
Inventor: **Hermolin, Joshua**
**14, Ha'Avoda Str.**
**Ramat-Hasharon 47445(IL)**
Inventor: **Feldman, David**
**18, Peretz Markish Str.**
**Haifa(IL)**
Inventor: **Zviely, Michael**
**3, Haim-Hazaz Str.**
**Savioney-Dania, Haifa 34984(IL)**
Inventor: **Zamir, Dov**
**Moshav Beerotalm**
**Doar Na Lev Hasharon(IL)**
Inventor: **Keselman, Hugo**
**1/3, Netiv Halotus St.**
**Karmiel(IL)**

(74) Representative: **Perani, Aurelio et al**
**c/o JACOBACCI-CASETTA & PERANI Via**
**Visconti di Modrone 7**
**I-20122 Milano(IT)**

(54) **Novel hydroxylated phenoxy compounds and process for their preparation.**

(57) Compounds of the formula:

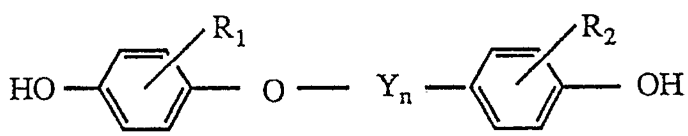

(I)

wherein:
n = 0 or 1;
$R_1$ and $R_2$ are $C_1$ to $C_3$ alkyl groups, or $R_2$ may be H when n = 0;
Y is selected from the group:

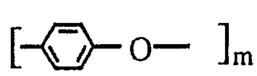 ; 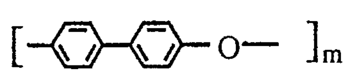

or

EP 0 446 722 A1

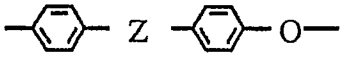

wherein:

m = 1-6;

Z = $SO_2$, S, C = O, $CH_2$ or $C(CH_3)_2$;

and a process for their preparation are described.

## Field of The Invention

The present invention relates to novel hydroxylated phenoxy compounds and to a process for the preparation of suoh compounds.

## Summary of The Invention

The compounds according to the invention have the formula:

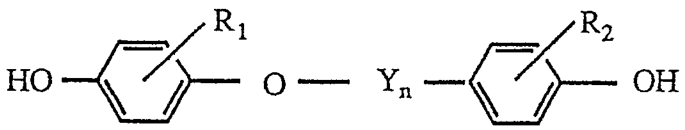

(I)

wherein:

n = 0 or 1;

$R_1$ and $R_2$ are $C_1$ to $C_3$ alkyl groups, or $R_2$ may be H when n = 0;

Y is selected from the group:

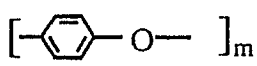

;

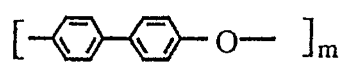

or

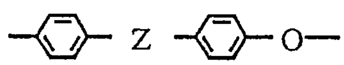

wherein:

m = 1-6;

Z = $SO_2$, S, C = O, $CH_2$ or C $(CH_3)_2$;

with the proviso that $R_1$ and $R_2$ are not both $CH_3$ when n = 0.

Illustrative, but non-limitative, compounds of formula I include:

4,4'-Dihydroxy-2-isopropyldiphenyl ether (DHIPE)
1,4-Bis (4'-hydroxy-3'-methylphenoxy) benzene (BHMMPZ)
1,4-Bis (4'-hydroxy-2'-methylphenoxy) benzene (BHMPZ)
4,4'-Bis (4"-hydroxy-2"-methylphenoxy) biphenyl (BHMPL)
4,4'-Bis (4"-hydroxy-3"-methylphenoxy) biphenyl (BHMMPL)
4,4'-Bis (4"-hydroxy-2"-methylphenoxy) diphenyl ether (BHMPDE)
4,4'-Bis (4"-hydroxy-3"-methylphenoxy) diphenyl ether (BHMMPDE)
4,4'-Bis (4"-hydroxy-2"-methylphenoxy) diphenyl sulfone (BHMPDSO)
4,4'-Bis (4"-hydroxy-3"-methylphenoxy) diphenyl sulfone (BHMMPDSO)
4,4'-Bis (4"-hydroxy-2"-methylphenoxy) diphenylmethane (BHMPDM)
4,4'-Bis (4"-hydroxy-3"-methylphenoxy) diphenylmethane (BHMMPDM)
4,4'-Bis (4"-hydroxy-2"-methylphenoxy) diphenyl sulfide (BHMPDS)
4,4'-Bis (4"-hydroxy-3"-methylphenoxy) diphenyl sulfide (BHMMPDS)
2,2-Bis [4'-(4"-hydroxy-2"-methylphenoxy) phenyl] propane (BHMPPP)
2,2-Bis [4'-(4"-hydroxy-3"-methylphenoxy) phenyl] propane (BHMMPPP)
4,4'-Bis (4"-hydroxy-2"-methylphenoxy) benzophenone (BHMPBP)
4,4'-Bis (4"-hydroxy-3"-methylphenoxy) benzophenone (BHMMPBP)

A specific compound of formula I, 4,4'-dihydroxy-3-methyl-diphenylether (DHMDPE) is known in the art from French Patent Publication No. 2 381 017, and is used, according to that patent application, as an intermediate for the preparation of pharmaceutical compounds. This compound is further useful as a precursor for a variety of polymers.

These 4,4'-disubstituted phenyl ethers are industrially useful monomers for the production of high performance polymers for application in the aviation, electrical, electronics and aerospace industries. Because of the many applications, a wide range of properties are sought by structural changes in the monomers. Thus, altering these monomers by substituents in the aromatic ring, and/or the substitution of one radical which serves to bind two of the aromatic rings by another, affects the softening point, flexibility, heat resistance, etc., of the resins produced therefrom. The improved properties they provide can benefit a wider range of applications which seek better performance from lighter-weight materials. The family of compounds described herein serve to provide this range of variability in the polymer properties.

The process known in the art for the preparation of DHMDPE, involves the Ullmann condensation of 4-methoxyphenol and 4-bromo-2-methylanisole, followed by dealkylation (demethylation in this specific case) of the di-ether formed. The process is relatively expensive, as it requires starting materials which are not readily available.

It is an object of the present invention to provide a simple process for the preparation of the compounds of formula I, including DHMDPE, which utilizes relatively inexpensive raw materials, and which is convenient and economic.

## Detailed Description of the Invention

The process according to the invention, by means of which compounds of formula I are prepared, comprises reacting a corresponding dibromo compound of formula II:

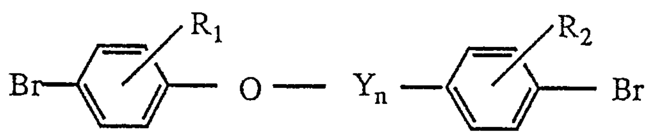

(II)

wherein $R_1$, $R_2$, Y and n have the meanings defined above, in a mixed solvent consisting of a basic aqueous medium and one or more alcohols, in the presence of a copper compound catalyst and at an elevated temperature.

The process of the invention will be illustrated in detail hereinafter, using the preparation of DHMDPE as the representative procedure. Other compounds of formula I are prepared in an analogous manner, using of course the starting material that is appropriate in each case, according to the compound that is desired to be obtained.

DHMDPE is prepared, according to the invention, starting from a readily available raw material, viz., m-phenoxytoluene (m-PHT) which is brominated to form 4,4'-dibromo-3-methyl-diphenylether (DBMDPE), followed by hydrolysis. DBMDPE is in itself a novel compound which is described and claimed in a copending patent application of the same applicant.

The aqueous medium is conveniently made alkaline by the addition of an alkali selected from the hydroxides and the oxides of Na and K, or of mixtures thereof, or of alkaline earth metals. According to a preferred embodiment of the invention, the alkali/DBMDPE molar ratio is in the range between 4-10.

According to another preferred embodiment of the invention, the alcohol is a lower alkyl alcohol selected from methanol, ethanol, propanol and butanol. Preferably, the alcohol:$H_2O$ ratio is from about 1:3 to about 3:1 by weight.

The copper compound catalyst is a compound of the formula

$Cu_nR_m$

wherein:

R is -O, -OH or a residue of an organic or an inorganic acid;

n is 1 or 2; and

m is 0,1 or 2.

Preferred copper compound catalysts are, for example, CuO, CuCl, $Cu(OAc)_2$, $Cu_2O$, $CuCl_2$, $CuBr_2$, $CuSO_4$ and CuBr. Preferably, one or more copper compound catalysts are employed, in a total amount of 0.1 to 10 % by mole DBMDPE, preferably between 2-7% by mole. A lower molar ratio of the catalyst leads to too slow reaction rates, while higher molar ratios are uneconomical, although they of course can be

employed.

The reaction can be carried out at a variety of pressures and temperatures, but too low temperatures lead to slow reaction rates, while excessively high temperatures lead to excessively high pressures requiring more expensive equipment. Therefore, the reaction is preferably carried out under autogenous pressure, at a temperature in the range 160° C-300° C. It should be noted that the reaction should be carried out under efficient stirring, which is needed for a fast and complete conversion of the starting material. Ineffective stirring may lead to incomplete and/or slow reactions.

Small quantities of impurities formed during the reaction, such as alkoxylated by-products and mono-hydrogenolized products, do not lead to technological complications as they can be easily removed from the aqueous basic product mixture by known techniques such as extraction with n-butanol or toluene. The residual aqueous solution is neutralized, the solvent stripped off and the crude product recrystallized.

## DETAILED DESCRIPTION OF PREFERRED EMBODIMENTS

### Brief Description of the Drawings

- Fig. 1 is the $^1$H-NMR spectrum of DHMDPE;
- Fig. 2 is the $^{13}$C-NMR spectrum of DHMDPE;
- Fig. 3 is the IR spectrum of DHMDPE;
- Fig. 4 is the GC/MS spectrum of DHMDPE;
- Fig. 5 is the mass spectrum of 1,4-bis (4'-hydroxy-3'-methylphenoxy) benzene (BHMMPZ);
- Fig. 6 is the mass spectrum of 1,4-bis (4'-hydroxy-2'-methylphenoxy) benzene (BHMPZ); and
- Fig. 7 is the NMR spectrum of BHMPZ; and
- Fig. 8 is GCMS analysis of 4,4'-bis (4"-hydroxy-2"-methylphenoxy) biphenyl after silylation.

The above and other characteristics and advantages of the invention will be better understood through the following illustrative and non-limitative description of preferred embodiments thereof. The analytical and spectral data were determined with the instruments and conditions detailed below.

GC

Gas-chromatograph - Varian 3400

| | |
|---|---|
| Oven: | Initial temp. 100° C, held 1 min., then raised to 250° C at 15° C/min. |
| Injector: | 250° C |
| Detector (transfer line): | 300° C |
| Column: | HP-1 (100% methyl polysiloxane), 5 m x 0.53 mm (megabore) |
| Injection amounts: | 0.5 μl |
| Flow: | 13 ml/min. |
| Retention time: | 7.62 min. |

GC/MS

Gas-chromatograph HP 5890A

| | |
|---|---|
| Oven: | Initial temp. 100° C, held 1 min., then raised to 240° C at 15° /min. |
| Injector: | 230° C |
| Detector (transfer line): | 250° C. |

Column: SUPELCO (fused silica capillary column) 30m x 0.25 mm)

| | |
|---|---|
| Split ratio: | 1:50 |
| Injection amounts: | 1 μl |
| Flow: | 0.6 ml/min. |
| Retention time: | 16.5 mins |

Mass Spectrometer HP 5970

| | |
|---|---|
| Range: | 40-550 a.m.n. |
| Scan: | every 0.9 sec. |

NMR spectra Bruker WP 200 MHz

| | $^1$H-NMR | $^{13}$C-NMR |
|---|---|---|
| Solvent: | DMSO-$d_6$ | DMSO-$d_6$ |
| Scans: | 50 | 10,000 |
| Reference: | TMS | TMS |

IR spectra

FTIR Nicolet 5MX

| | |
|---|---|
| Range: | 400-4600 $cm^{-1}$ |
| Scan: | 10 (every 1.0 sec.) |
| Sample: | 0.8 mg/80 mg KBr |

**Example 1**

**A) Preparation of DBMDPE**

The starting material, 4,4'-dibromo-3-methyldiphenylether (DBMDPE) was prepared as follows. To a four-necked round-bottomed flask equipped with a stirrer, a condenser, a dropping funnel and a thermometer, containing a stirred solution of m-PHT (460 g, 2.5 moles) in one liter of dichloromethane, there were added 840 g ( 5.25 moles) of $Br_2$. The top of the condenser was equipped with a trap to absorb HBr released during the reaction. $Br_2$ was added at a temperature between - 5° and 0° C and in complete darkness, during one hour. After the addition, the reaction mixture was stirred for an additional 1 hour at about 25° C. The progress of the reaction was determined by GC. Excess bromine and traces of HBr were neutralized with aqueous 10% $NH_3$ (150 ml). Two phases formed in the reaction, which were separated, and the organic layer was washed with water (200 ml). After distillation of the solvent in the organic phase, DBMDPE (820 g) was obtained, containing 97% of the desired isomer (determined by GC) which represents a yield of 93%.

After crystallization from methanol, a product of 99% purity was obtained, containing 46.4% Br, and having a melting point of 44-46° C. The crystallization from ethanol gave identical results.

The product so obtained was characterized by GC under the conditions described above, which showed a main peak (99.6%) at a retention time of 7.05 min. The structure of the compound was confirmed by Mass Spectra and NMR, as described in the copending Israeli patent application No. 93684 of the same applicant herein, the specification of which is incorporated herein by reference.

**B) Hydrolysis of 4,4'-Dibromo-3-methyl-diphenylether DBMDPE)**

Into a 1 liter 316 stainless steel autoclave, there was placed DBMDPE (171.0 g, 0.5 moles), 30% aqueous NaOH (400 g, 3.0 moles), ethanol (300 ml) and CuCl (2.0 g, 0.02 moles). The autoclave was sealed and heated to 200° C. Full conversion was achieved after one hour. The autoclave was cooled and the pressure released, then opened. The reaction mixture was filtered to recover the catalyst, then most of the ethanol was removed by distillation. The mixture was then neutralized to pH 5 with concentrated HCl and the products were extracted with ethyl acetate.

The solvent was then removed to give 102 g reaction product containing 80% DHMDPE (by NMR

analysis).

In order to achieve a pure product directly, the original, basic reaction mixture was extracted with toluene to remove alkoxylated by-products, then the aqueous reaction mixture was neutralized with HCl and the work-up continued as above to give 75 g pure (99%) DHMDPE with a m.p. 112-113 °C.

GC shows a major peak at 7.62 mins. Fig. 1 is the $^1$H-NMR of DHMDPE, Fig. 2 is the $^{13}$C-NMR, Fig. 3 is the IR and Fig. 4 is the GC/MS spectrum.

## Example 2

Example 1B was repeated using methanol instead of ethanol and $Cu(OAc)_2$ as catalyst. Pure DHMDPE was obtained in a yield of 60%.

## Examples 3-8

In order to illustrate the effect of reaction temperature and of the mixed solvent, the reaction of Example 1B was repeated under different conditions, as shown in Table I below. It is easily seen that low reaction temperatures, as well as too low ethanol content, adversely affect the conversion.

### Table 1

| | Reagents (g) | | | | Temp. | Time | Conversion |
|---|---|---|---|---|---|---|---|
| Ex. No. | DBMDPE | 30% NaOH | EtOH | CuCl | °C | hrs. | % |
| 3 | 68.4 | 160.0 | 120.0 | 2.0 | 200 | 1.0 | 99.0 |
| 4 | 68.4 | 160.0 | 120.0 | 2.0 | 170 | 2.0 | 36.0 |
| 5 | 68.4 | 160.0 | 120.0* | 2.0 | 180 | 4.0 | 98.0 |
| 6 | 68.4 | 160.0 | 120.0 | 2.0 | 200 | 3.0 | 99.0 |
| 7 | 68.4 | 160.0 | - | 2.0 | 200 | 2.0 | <5.0 |
| 8 | 68.4 | 160.0 | 30.0 | 2.0 | 200 | 2.0 | 15.0 |

*n-Butanol was used instead of EtOH.

## Examples 9-12

Example 1B was repeated using different copper compound catalysts, as follows:
1) CuO; 2) CuBr; 3) $CuSO_4 \cdot 5H_2O$; 4) $Cu_2O$ recycled from a previous reaction.

The results obtained were in all cases similar to those obtained in Example 1B. It should be noted that the ability to recycle the catalyst influences the production costs and is an important factor in industrial production.

## Example 13

**1,4-Bis (4'-hydroxy-3'-methylphenoxy) benzene (BHMMPZ)**

Into a 1 liter SS-316 Parr autoclave reactor were introduced: 1,4-Bis (4'-bromo-3'-methylphenoxy) benzene (9.9 g, 0.022 moles); aqueous 6N NaOH (250 ml, 1.5 moles); ethanol (200 ml) and CuCl (0.22 g, 0.0022 moles).

The mixture was stirred at 220 °C (690-630 psi) for 3 hours with rapid stirring (~1200 rpm). After the reaction completion, the autoclave was cooled to room temperature, opened, and its content was transferred to a 1-liter flask. The reaction mixture was stirred for an additional hour under reflux conditions in the presence of active carbon (2 g). After filtration, the filtrate was brought to pH 1 with conc. HCl and the crude product was filtered by suction. 6 G of crude product were obtained. GCMS analysis of the crude mixture showed 60% of BHMMPZ. The mass spectrogram of a purified sample is shown in Fig. 5. Acid/base treatment and recrystallization gave BHMMPZ of the following analysis: 74.4% C, 5.6% H. Calc'd: 74.5% C and 5.6% H.

## Example 14

### 1,4-Bis (4'-hydroxy-2'-methylphenoxy) benzene (BHMPZ)

Into a 1 liter SS-316 Parr autoclave reactor were introduced: 1,4-Bis (4'-bromo-2'-methylphenoxy) benzene (22.4 g, 0.05 moles); aqueous 6N NaOH (250 ml, 1.5 moles); ethanol (250 ml) and CuCl (0.49 g, 0.005 moles).

The mixture was stirred at 200 °C (430-410 psi) for two hours with rapid stirring (~1200 rpm). After the reaction completion, the reaction mixture was filtered by suction and the ethanol was removed by vacuum evaporation. The aqueous phase was brought to pH 5 with conc. HCl and extracted with ethyl acetate (300 ml). After removal of the solvent, 16.8 g of crude oily product was obtained. GCMS analysis of the crude mixture showed 34.6% of BHMPZ. Acid/base treatment and recrystallization afforded BHMPZ with 97% purity (GC). The mass spectrogram is shown in Fig. 6, and the NMR spectrum is in Fig. 7.

## Example 15

### 4,4'-Dihydroxy-2-isopropyldiphenyl ether (DHIPE)

Into a 1 liter SS-316 Parr autoclave reactor were introduced: 4,4'-dibromo-2-isopropyldiphenyl ether (3.7 g, 0.01 moles); aqueous 6N NaOH (250 ml, 1.5 moles); ethanol (200 ml) and CuCl (0.3 g, 0.003 moles).

The mixture was stirred at 200-220 °C (440-620 psi) for 8 hours with rapid stirring (~1200 rpm). After the reaction completion, the autoclave was cooled to room temperature, opened and its content was transferred to a 1 liter flask. The reaction mixture was stirred for an additional hour under reflux conditions in the presence of active carbon (1 g). After filtration, the ethanol was removed by vacuum evaporation. The aqueous phase was brought to pH 1 with conc. HCl and extracted with ethyl acetate (100 ml). After removal of the solvent 1.9 g of crude oily product were obtained. GCMS analysis of the crude mixture showed 51% of DHIPE. Acid/base treatment and recrystallization gave DHIPE of the following analysis: 73.5% C and 6.6% H. Calc'd: 73.8% C and 6.5% H.

## Example 16

### 4,4'-Bis (4"-hydroxy-2"-methylphenoxy) biphenyl (BHMPL)

Into a 1 liter SS-316 Parr autoclave reactor were introduced: 4,4'-Bis (4"-bromo-2"-methylphenoxy) biphenyl (2.1 g, 0.004 moles); aqueous 6N NaOH (250 ml, 1.5 moles); ethanol (200 ml) and CuCl (0.34 g, 0.0017 moles).

The mixture was stirred at 210 °C (580 psi) for 4 hours with rapid stirring (~1200 rpm). After the reaction completion, autoclave was cooled to room temperature, opened and its content was transferred to a one liter flask. The reaction mixture was stirred for an additional hour under reflux conditions in the presence of active carbon (1 g). After filtration, the ethanol was removed by vacuum evaporation. The aqueous phase was brought to pH 1 with conc. HCl and extracted with ethyl acetate (150 ml). After removal of the solvent, 1.5 g of crude product were obtained. GCMS analysis of the crude mixture after silylation showed 89% of BHMPL (Fig. 8).

## Example 17

**4,4'-Bis (4"-hydroxy-2"-methylphenoxy) diphenylmethane (BHMPDM)**

In a manner similar to Example 13, BHMPDM was made from 15 g of 4,4'-bis- (4"-bromo-2"-methylphenoxy) diphenylmethane. A product of the following analysis was obtained: 78.7% C, 5.9% H; calc'd: 78.6% and 5.8% H.

**Example 18**

**4,4'-Bis (4"-hydroxy-2"-methylphenoxy) diphenylether (BHMPDE)**

In a manner similar to that of Example 13, BHMPDE was made from 22 g of 4,4'-bis (4"-bromo-2"-methylphenoxy) diphenylether. A product of the following analysis was obtained: 75.5% C, 5.4% H; calc'd: 75.4% C, 5.3% H.

## Claims

1. A compound of the formula:

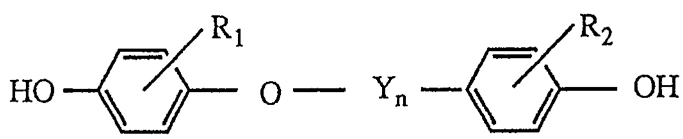

(I)

wherein:
n = 0 or 1;
$R_1$ and $R_2$ are $C_1$ to $C_3$ alkyl groups, or $R_2$ may be H when n = 0;
Y is selected from the group:

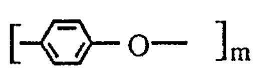 ; 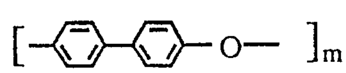

or

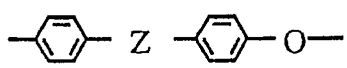

wherein:
m = 1-6;
Z = $SO_2$, S, C = O, $CH_2$ or $C(CH_3)_2$;
with the proviso that $R_1$ and $R_2$ are not both $CH_3$ when n = 0.

2. 4,4'-Dihydroxy-2-isopropyldiphenyl ether.

3. 1,4-Bis (4'-hydroxy-3'-methylphenoxy) benzene.

4. 1,4-Bis (4'-hydroxy-2'-methylphenoxy) benzene.

5. 4,4'-Bis (4"-hydroxy-2"-methylphenoxy) biphenyl.

6. 4,4'-Bis (4"-hydroxy-2"-methylphenoxy) diphenyl ether.

7. 4,4'-Bis (4"-hydroxy-2"-methylphenoxy) diphenyl sulfone.

**8.** 4,4'-Bis (4''-hydroxy-2''-methylphenoxy) diphenylmethane.

**9.** 4,4'-Bis (4''-hydroxy-2''-methylphenoxy) diphenyl sulfide.

**10.** 2,2'-Bis [4'-(4''-hydroxy-2''methylphenoxy) phenyl] propane.

**11.** 4,4'-Bis (4''-hydroxy-2''-methylphenoxy) benzophenone.

**12.** A process for preparing a compound of formula I, as defined in claim 1, comprising reacting a compound of the formula:

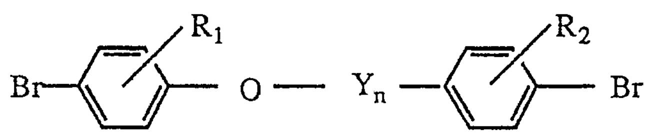

(II)

wherein $R_1$, $R_2$, Y and n are as defined in claim 1, in a mixed solvent consisting of a basic aqueous medium and one or more alcohols, in the presence of a copper compound catalyst and at an elevated temperature.

**13.** A process according to claim 12, wherein the aqueous medium is made alkaline by the addition of an alkali selected from the hydroxides and the oxides of Na and K, or mixtures thereof, or of alkaline earth metals.

**14.** A process according to claim 12 or 13, wherein the alcohol is a lower alkyl alcohol selected from methanol, ethanol, propanol and butanol.

**15.** A process according to any one of claims 12 to 14, wherein the alcohol:$H_2O$ ratio is from about 1:3 to about 3:1 by weight.

**16.** A process according to any one of claims 12 to 15, wherein the molar ratio of the alkali to the compound of formula II is in the range between 4-10.

**17.** A process according to any one of claims 12 to 16, wherein the copper compound catalyst is a compound of the formula

$Cu_nR_m$

wherein:
R is -O, -OH or a residue of an organic or inorganic acid;
n is 1 or 2; and
m is 0,1 or 2.

**18.** A process according to claim 17, wherein the copper compound catalyst is selected from among CuO, CuCl, $Cu(OAc)_2$, $Cu_2O$, $CuCl_2$, $CuBr_2$, $CuSO_4$ and CuBr.

**19.** A process according to claim 17 or 18, wherein one or more copper compound catalyst(s) are employed, in an amount of 0.1 to 10% by mole of the compound of formula II, preferably between 2% and 7% by mole.

**20.** A process according to any one of claims 12 to 19, wherein the reaction is carried out at a temperature in the range 160°C to 300°C.

**21.** A process according to any one of claims 12 to 20, wherein the copper compound catalyst is filtered off from the product mixture and, optionally, recycled to a further reaction.

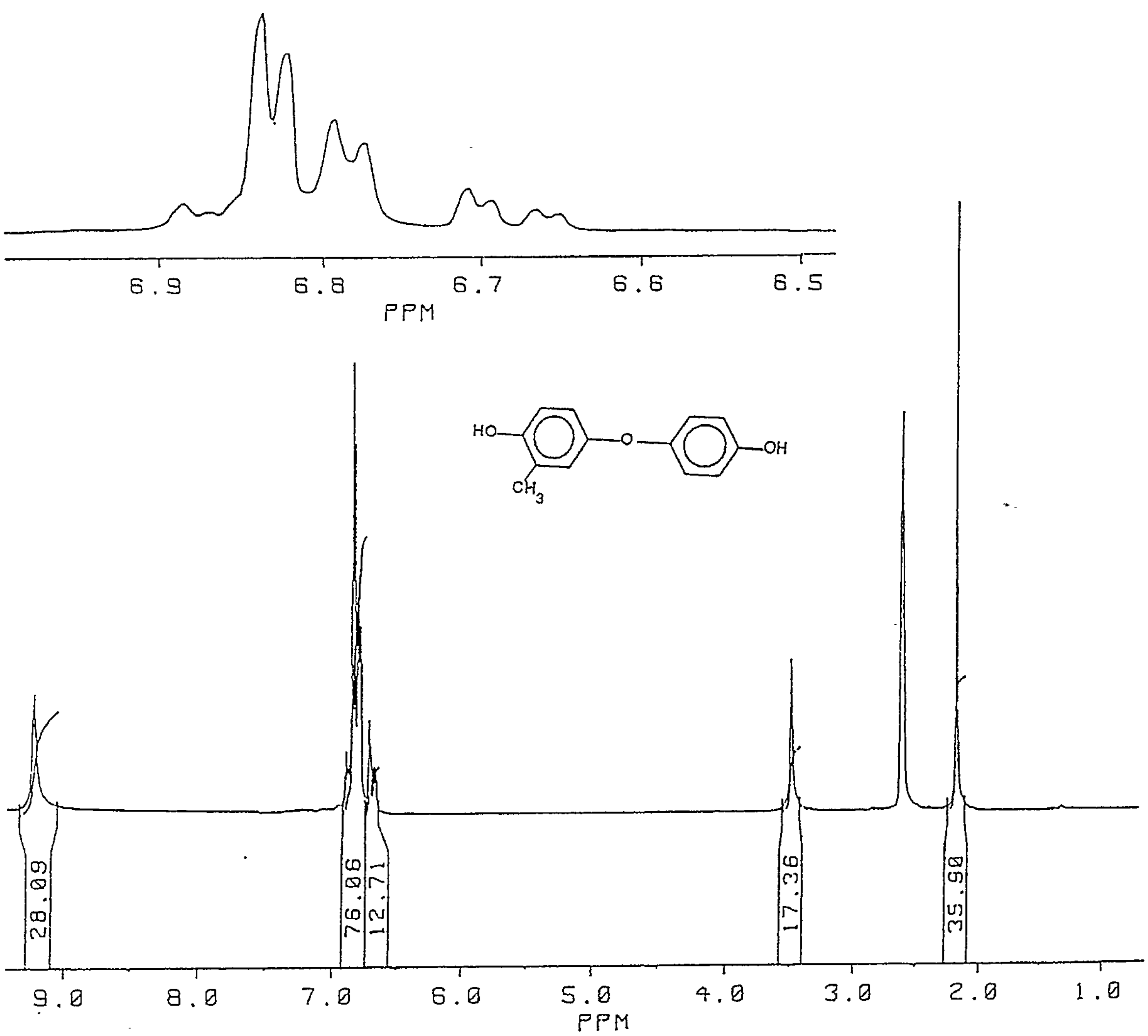
6.9
6.8
6.7
6.6
6.5
PPM
28.09
76.06
12.71
17.36
35.90
9.0
8.0
7.0
6.0
5.0
4.0
3.0
2.0
1.0
PPM

**Fig. 1**

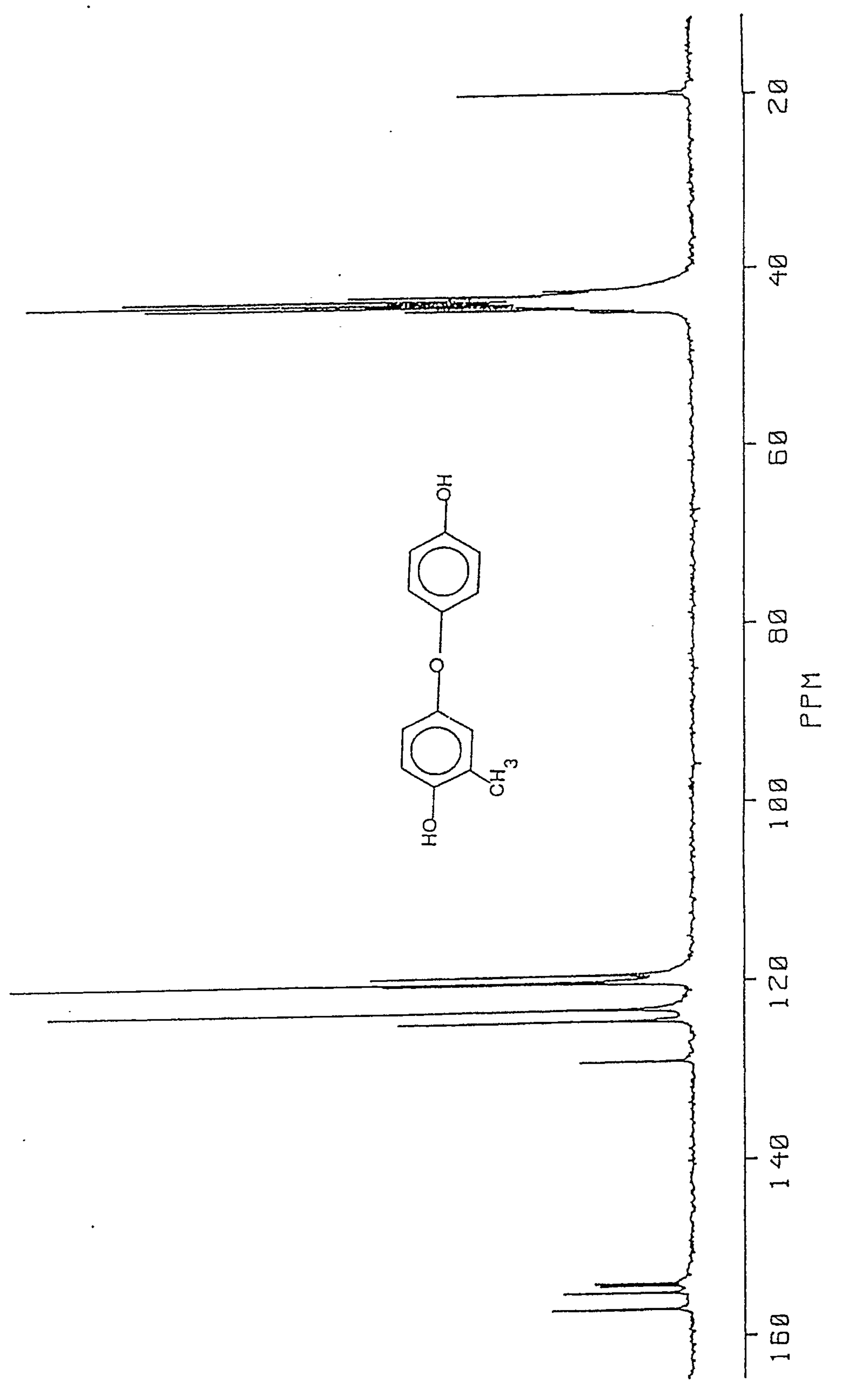


**Fig. 2**

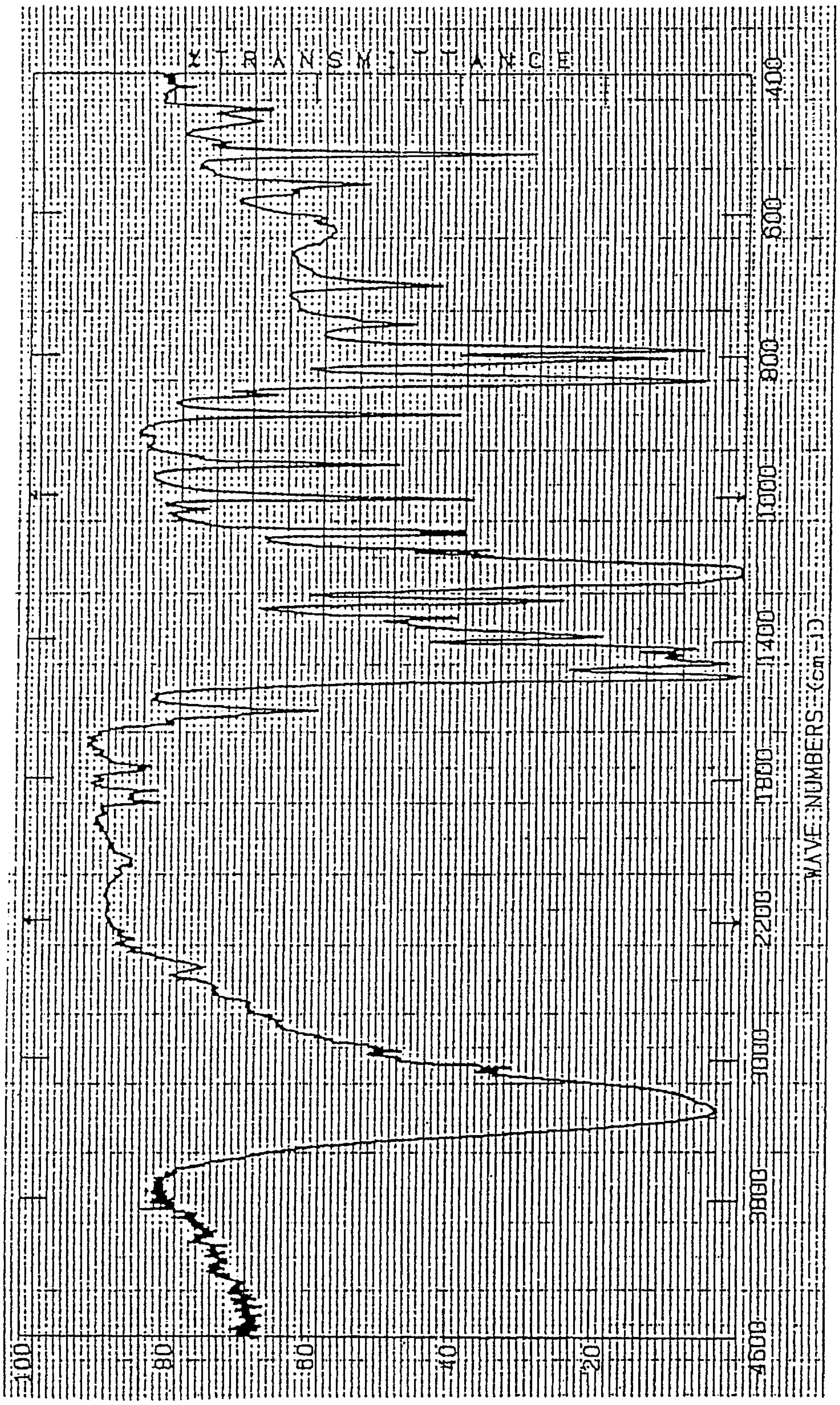
% TRANSMITTANCE
WAVE NUMBERS (cm-1)
100
80
60
40
20
4000
2200
1800
1400
1000
800
600
400

Fig. 3

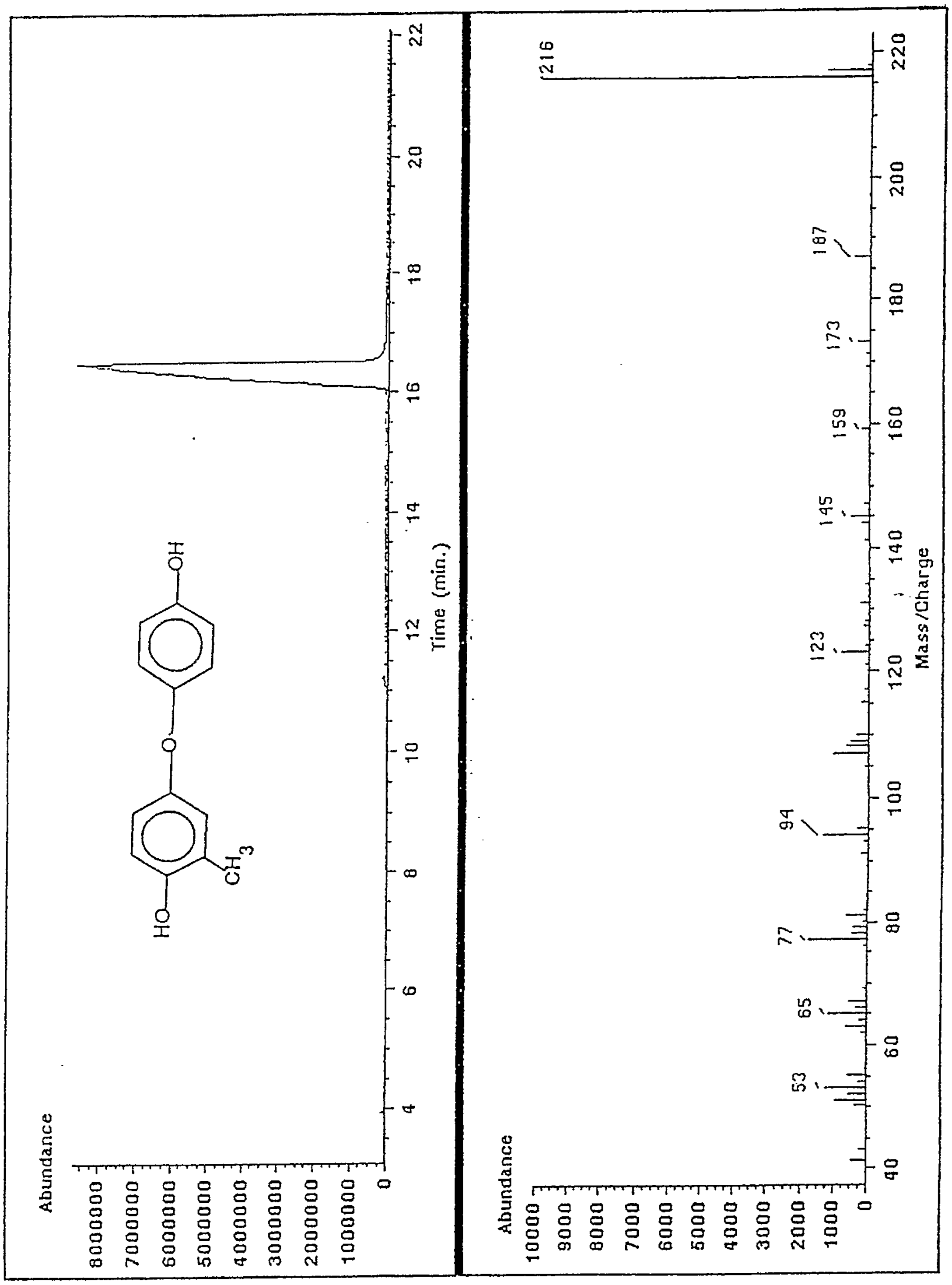
Abundance
8000000
7000000
6000000
5000000
4000000
3000000
2000000
1000000
0
4
6
8
10
12
14
16
18
20
22
Time (min.)
Abundance
10000
9000
8000
7000
6000
5000
4000
3000
2000
1000
0
40
60
80
100
120
140
160
180
200
220
Mass/Charge
53
65
77
94
123
145
159
173
187
216

Fig. 4

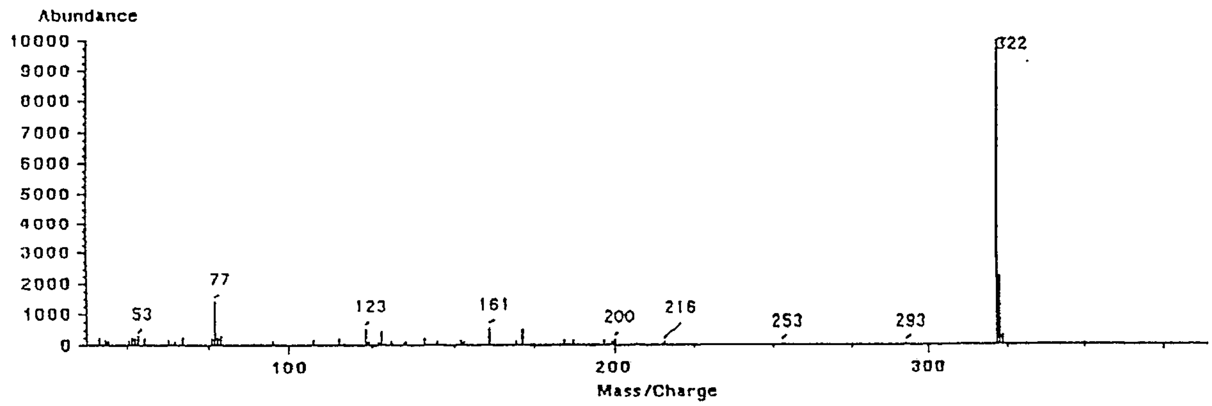


Fig. 5

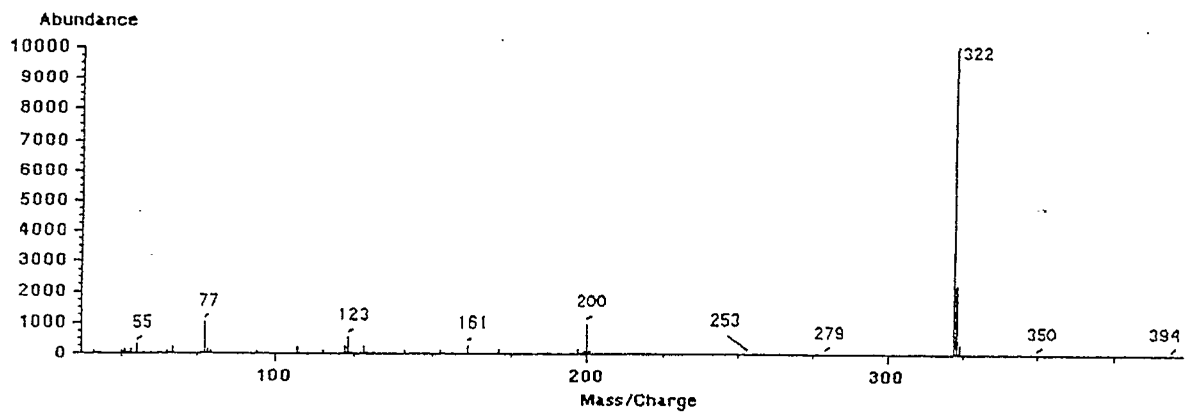


Fig. 6

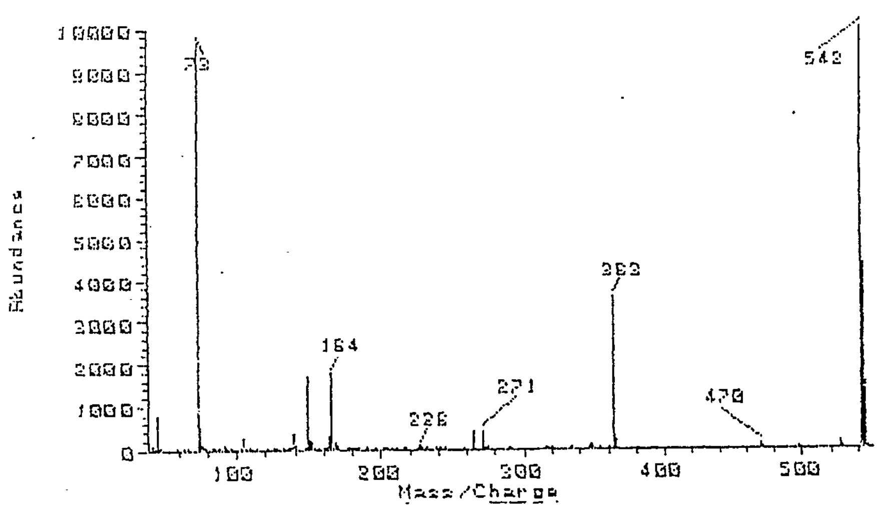


Fig. 8

Fig. 7

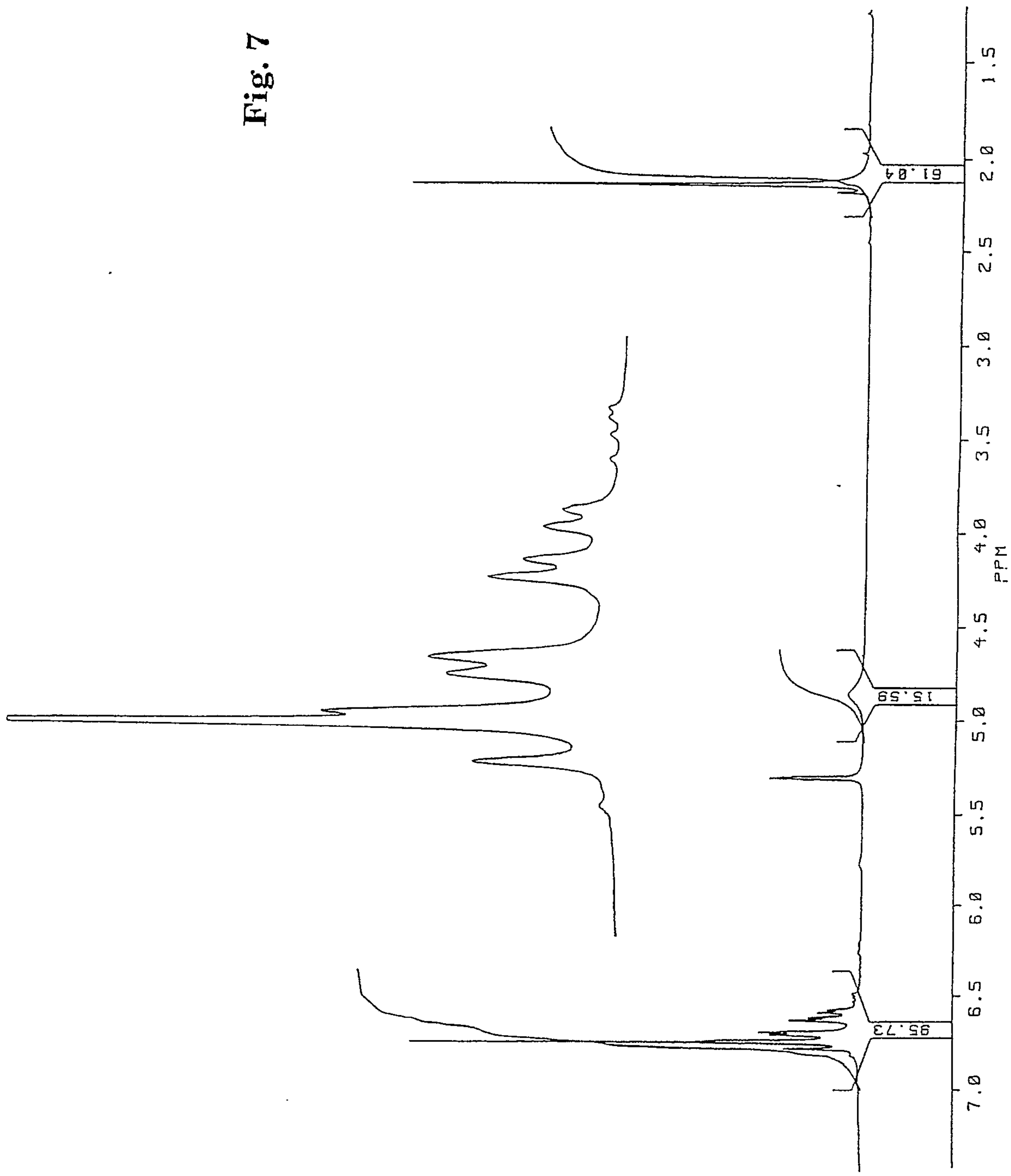

European Patent Office

# EUROPEAN SEARCH REPORT

Application Number

**EP 91 10 3085**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | FR-A-2 381 017 (ALBERT ROLLAND)<br>* Example 7A * | 1 | C 07 C 43/295<br>C 07 C 49/84<br>C 07 C 317/22<br>C 07 C 323/20 |
| A | US-A-3 290 386 (G.S. STAMATOFF)<br>* Complete document * | 1,12-21 | |
| A | EP-A-0 122 412 (BAYER)<br>* Claims; examples * | 1 | |
| A | US-A-4 306 094 (R.J. SHOZDA)<br>* Column 1, lines 23-28; example 12 * | 1 | |
| A | CA-A-1 051 146 (E.I. DU PONT DE NEMOURS)<br>* Pages 1,2 * | 1 | |
| | | | **TECHNICAL FIELDS SEARCHED (Int. Cl.5)** |
| | | | C 07 C 43/00<br>C 07 C 49/00<br>C 07 C 317/00<br>C 07 C 323/00 |

The present search report has been drawn up for all claims

| Place of search | Date of completion of search | Examiner |
|---|---|---|
| The Hague | 10 June 91 | WRIGHT M.W. |

**CATEGORY OF CITED DOCUMENTS**

X : particularly relevant if taken alone
Y : particularly relevant if combined with another document of the same catagory
A : technologioal background
O : non-written disclosure
P : intermediate document
T : theory or principle underlying the invention

E : earlier patent document, but published on, or after the filing date
D : document cited in the application
L : document cited for other reasons

& : member of the same patent family, corresponding document